# EUROPEAN PATENT APPLICATION

(11) **EP 2 135 541 A1**
(43) Date of publication of application: **23.12.2009**
(21) Application number: 08739342.7
(22) Date of filing: 28.03.2008
(51) Int. Cl.: A61B 1/00, A61B 1/04, A61B 5/07

(54) **CAPSULE TYPE MEDICAL DEVICE AND METHOD FOR MANUFACTURING THE SAME**

(30) Priority: 30.03.2007 JP 2007094891
(71) Applicant: Olympus Medical Systems Corp., Tokyo 151-0072 (JP)
(72) Inventor: SEGAWA, Hidetake, Tokyo 151-0072 (JP); ORIHARA, Tatsuya, Tokyo 151-0072 (JP)
(74) Representative: von Hellfeld, Axel
(86) International application number: PCT/JP2008/056224
(87) International publication number: WO 2008/120762

(57) **Abstract**

An object of the present invention is that a lens frame of an optical unit that forms images of inside a subject on a light receiving surface of an imaging unit can be easily inserted into an opening part of an illuminating board having an illuminating unit that illuminates the inside of the subject mounted thereon. A capsule medical device according to the present invention includes optical units 4 and 7 that form the images of inside the subject onto light receiving surfaces of solid-state imaging devices 5 and 8, respectively, and illuminating boards 19a and 19f having opening parts, respectively, for inserting lens frames 4d and 7d of the optical units 4 and 7 thereinto formed thereon, respectively. The lens frames 4d and 7d each have a tapered external shape with an upper end being tapered, and the lens frames are inserted into the opening parts of the illuminating boards 19a and 19f from the tapered upper ends, respectively.

## Description

### TECHNICAL FIELD

The present invention relates to a capsule medical device introduced into internal organs of a subject such as a patient to acquire in-vivo information of the subject, and a method of manufacturing a capsule medical device.

### BACKGROUND ART

Conventionally, in the field of endoscope, a swallowing-type capsule endoscope having an imaging function and a wireless communication function has been proposed. The capsule endoscope is introduced into internal organs by swallowing it from a mouth of the subject such as a patient for observation (examination) of the internal organs. Thereafter, the capsule endoscope moves in the internal organs with peristaltic movements or the like, while sequentially capturing images of inside of the subject (hereinafter, occasionally "in-vivo images") at a predetermined interval, for example, at an interval of 0.5 second, and finally, it is naturally discharged to the outside of the subject.

The in-vivo images captured by the capsule endoscope while the capsule endoscope is present inside the internal organs of the subject are sequentially transmitted from the capsule endoscope to an external receiving device by wireless communication. The receiving device is carried by the subject to receive an in-vivo image group wirelessly transmitted from the capsule endoscope introduced into the internal organs of the subject, and stores the received in-vivo image group on a recording medium.

The in-vivo image group stored on the recording medium of the receiving device is taken in an image display device such as a workstation. The image display device displays the in-vivo image group of the subject acquired via the recording medium. A doctor, a nurse or the like can diagnose the subject by observing the in-vivo image group displayed on the image display device.

For example, as disclosed in Patent Documents 1 and 2, such a capsule endoscope has a capsule casing with a transparent optical dome at an end, and includes, inside the capsule casing, an illuminating unit such as an LED that illuminates inside of the internal organs over the optical dome, an optical unit that forms reflected light from inside of the internal organs illuminated by the illuminating unit, and an imaging unit such as a CCD that captures images of the inside of the internal organs (that is, in-vivo images) formed by the optical unit. The illuminating unit and the imaging unit are incorporated in the capsule casing in a manner in which they are mounted on an illuminating board and an imaging board, respectively, which are rigid circuit boards (hereinafter, simply "rigid board"). The illuminating board and the imaging board are electrically connected with each other via a flexible circuit board (hereinafter, simply "flexible board").

Patent Document 1: Japanese Patent Application Laid-open No. 2005-198964
Patent Document 2: Japanese Patent Application Laid-open No. 2005-204924

### DISCLOSURE OF INVENTION

### PROBLEM TO BE SOLVED BY THE INVENTION

The optical unit incorporated in the conventional capsule endoscope has a lens group that collects reflected light from the inside of the internal organs illuminated by the illuminating unit on the illuminating board, and a cylindrical lens frame that holds the lens group. The lens group held by the lens frame forms images of the inside of the internal organs illuminated over the optical dome by the illuminating unit on the illuminating board onto a light receiving surface of the imaging unit. To realize this, a lower end of the lens frame is fixed with respect to the imaging unit, with a lower end of the lens group facing the light receiving surface of the imaging unit. The lens frame with the lower end thereof being fixed with respect to the imaging unit is inserted into an opening part of the illuminating board, in a manner in which an upper end of the lens group faces the optical dome.

However, when the cylindrical lens frame is inserted into the opening part of the illuminating board, alignment between the opening part of the illuminating board and an outer circumference of the upper end of the lens frame takes time and labor, thereby causing a problem that the insertion of the lens frame into the opening part of the illuminating board is difficult.

The present invention has been achieved in view of such circumstances, and an object of the present invention is to provide a capsule medical device, in which a lens frame of an optical unit that forms images of inside a subject onto a light receiving surface of an imaging unit can be easily inserted into an opening part of an illuminating board having an illuminating unit that illuminates the inside of the subject mounted thereon, and a method of manufacturing a capsule medical device.

### MEANS FOR SOLVING PROBLEM

To solve the problem described above and achieve the object, a capsule medical device according to the present invention is introduced into a subject, and includes an illuminating unit that illuminates inside of the subject and an imaging unit that captures an image of inside the subject illuminated by the illuminating unit. The capsule medical device includes an optical unit including a lens group that forms the image of inside the subject illuminated by the illuminating unit onto a light receiving surface of the imaging unit and a lens frame for holding the lens group; and an illuminating board having an opening part for inserting the lens frame thereinto formed thereon and having the illuminating unit mounted thereon. The lens frame has a tapered external shape with one end being tapered, and is inserted into the opening part of the illuminating board from the one end being tapered.

In the capsule medical device according to the present invention, the illuminating board has a bending part extending to a part of an outer circumference, and turns in a direction of moving the opening part toward the lens frame by bending the bending part. The lens frame has the tapered external shape capable of being inserted into the opening part while avoiding a contact between the illuminating board, which turns while bending the bending part, and the one end.

In the capsule medical device according to the present invention, the lens frame has the tapered external shape, with a distal side with respect to the bending part being inclined.

A capsule medical device according to the present invention is introduced into a subject, and includes an illuminating unit that illuminates inside of the subject and an imaging unit that captures an image of inside the subject illuminated by the illuminating unit. The capsule medical device includes an optical unit including a lens group that forms the image of inside the subject illuminated by the illuminating unit onto a light receiving surface of the imaging unit and a lens frame for holding the lens group; and an illuminating board having a bending part extending to a part of an outer circumference, and an opening part in which an aperture capable of inserting the lens frame of the optical unit thereinto is opened and enlarged toward a distal side with respect to the bending part formed thereon, and having the illuminating unit mounted thereon. The illuminating board turns in a direction of moving the opening part toward the lens frame by bending the bending part. The lens frame is inserted into the opening part while avoiding a contact between the illuminating board, which turns while bending the bending part, and an upper end of the lens frame.

A capsule medical device according to the present invention is introduced into a subject, and includes an illuminating unit that illuminates inside of the subject and an imaging unit that captures an image of inside the subject illuminated by the illuminating unit. The capsule medical device includes an optical unit including a lens group that forms the image of inside the subject illuminated by the illuminating unit onto a light receiving surface of the imaging unit and a lens frame for holding the lens group; and an illuminating board having a bending part extending to a part of an outer circumference and an opening part capable of inserting the lens frame thereinto formed thereon, and having the illuminating unit mounted thereon. At least one of the opening part and the lens frame has a shape capable of avoiding a contact between the illuminating board, which turns while bending the bending part, and an upper end of the lens frame.

In the capsule medical device according to the present invention, the shape capable of avoiding the contact is a tapered external shape with one end of the lens frame being tapered.

In the capsule medical device according to the present invention, the shape capable of avoiding the contact is an opening shape in which the opening part of the illuminating board is opened and enlarged toward a distal side with respect to the bending part.

A capsule medical device according to the present invention is introduced into a subject, and includes an illuminating unit that illuminates inside of the subject and an imaging unit that captures an image of inside the subject illuminated by the illuminating unit. The capsule medical device includes an optical unit including a lens group that forms the image of inside the subject illuminated by the illuminating unit onto a light receiving surface of the imaging unit and a lens frame for holding the lens group; and an illuminating board having a bending part extending to a part of an outer circumference and an opening part capable of inserting the lens frame thereinto formed thereon, and having the illuminating unit mounted thereon. The illuminating board, which turns while bending the bending part, turns in a direction of moving the opening part toward an upper end of the lens frame. The capsule medical device has a shape capable of inserting the lens frame into the opening part while avoiding a contact between the opening part and the upper end of the lens frame.

In the capsule medical device according to the present invention, the shape capable of inserting the lens frame into the opening part while avoiding the contact is formed of the lens frame having a tapered external shape with an upper end being tapered; and the opening part of the illuminating board having an opening shape opened and enlarged toward a distal side with respect to the bending part.

In the capsule medical device according to the present invention, the illuminating board is a flexible circuit board.

A method of manufacturing a capsule medical device according to the present invention includes a step of bending an illuminating board having an opening part for inserting a lens frame thereinto formed thereon, and a bending part extending to a part of an outer circumference thereof; and a step of inserting the lens frame having a tapered external shape with one end being tapered into the opening part while avoiding a contact between the illuminating board and the lens frame.

A method of manufacturing a capsule medical device according to the present invention includes a step of bending an illuminating board having a bending part extending to a part of an outer circumference thereof and an opening part, which is opened and enlarged toward a distal side with respect to the bending part, for inserting a lens frame thereinto; and a step of inserting the lens frame into the opening part while avoiding a contact between the illuminating board and the lens frame.

### EFFECT OF THE INVENTION

In the capsule medical device according to the present invention, an external shape of the lens frame of the optical unit that forms images of inside the subject illuminated by the illuminating unit onto the light receiving surface of the imaging unit is tapered, an opening part capable of inserting the lens frame thereinto is formed in the illuminating board having the illuminating unit mounted thereon, and the lens frame is inserted into the opening part of the illuminating board from a tapered end of the lens frame. Therefore, when the lens frame is inserted into the opening part of the illuminating board, the opening part and the outer circumference of the upper end of the lens frame can be easily aligned with each other, and a contact between the upper end of the lens frame and the illuminating board can be avoided. As a result, there is an operational effect such that, the lens frame of the optical unit that forms the images of inside the subject onto the light receiving surface of the imaging unit can be easily inserted into the opening part of the illuminating board having the illuminating unit that illuminates the inside of the subject mounted thereon.

Further, in the capsule medical device according to the present invention, an opening part, in which an aperture capable of inserting the lens frame of the optical unit thereinto is opened and enlarged toward a distal side with respect to a bending part on an outer circumference of the illuminating board, is formed in the illuminating board, and the illuminating board turns around the bending part in a direction of moving the opening part toward the lens frame, thereby inserting the lens frame into the opening part of the illuminating board. Therefore, even if the lens frame has a cylindrical external shape, the opening part and the outer circumference of the upper end of the lens frame can be easily aligned with each other at the time of inserting the lens frame into the opening part of the illuminating board, and a contact between the upper end of the lens frame and the illuminating board can be avoided. As a result, there is an operational effect such that the lens frame of the optical unit that forms the images of inside the subject onto the light receiving surface of the imaging unit can be easily inserted into the opening part of the illuminating board having the illuminating unit that illuminates the inside of the subject mounted thereon.

### BRIEF DESCRIPTION OF DRAWINGS

FIG. 1 is a schematic longitudinal cross section of a configuration example of a capsule endoscope according to an embodiment of the present invention.
FIG. 2 is a schematic diagram for exemplifying an internal structure of the capsule endoscope as viewed over an optical dome from a direction F shown in FIG. 1.
FIG. 3 is a schematic diagram for exemplifying the internal structure of the capsule endoscope as viewed over the optical dome from a direction B shown in FIG. 1.
FIG. 4 is a schematic diagram for exemplifying a state where circuit components of a power supply system are mounted on a control board.
FIG. 5 is a schematic diagram for exemplifying a state where a series of circuit boards folded and arranged in a casing of the capsule endoscope is developed.
FIG. 6 is a schematic diagram for explaining fitting of a lens frame fitted to a plate-like portion of a positioning unit and an illuminating board.
FIG. 7 is a schematic diagram for explaining fitting of a lens frame having a cylindrical shape and the illuminating board.
FIG. 8 is a schematic longitudinal cross section of a configuration example of a capsule endoscope according to a second embodiment of the present invention.
FIG. 9 is a schematic diagram for exemplifying an internal structure of the capsule endoscope as viewed over an optical dome from a direction F shown in FIG. 8.
FIG. 10 is a schematic diagram for exemplifying the internal structure of the capsule endoscope as viewed over the optical dome from a direction B shown in FIG. 8.
FIG. 11 is a schematic diagram for explaining fitting of a lens frame fitted to a plate-like portion of a positioning unit and an illuminating board.
FIG. 12 is a schematic cross section of a configuration example of a capsule endoscope having lens frames having a tapered external shape only on one side of the outer circumference.

### EXPLANATIONS OF LETTERS OR NUMERALS

- 1, 21: Capsule endoscope
- 2: Casing
- 2a: Cylindrical body
- 2b, 2c: Optical dome
- 3a to 3d, 6a to 6d: Light-emitting element
- 4, 7, 24, 27: Optical unit
- 4a, 4b, 7a, 7b: Lens
- 4c, 7c: Aperture unit
- 4d, 7d, 24d, 27d: Lens frame
- 5, 8: Solid-state imaging device
- 9a: Wireless unit
- 9b: Antenna
- 10: Control unit
- 11a: Magnetic switch
- 11b, 11c: Capacitor
- 11d: Power supply IC
- 12a, 12b: Battery
- 13a, 13b: Contact spring
- 14, 15: Positioning unit
- 14a, 15a: Plate-like portion
- 14b, 15b: Protrusion
- 16, 17, 36: Load receiving unit
- 18a, 18b: Power supply board
- 19a, 19f, 29a, 29f: Illuminating board
- 19b, 19e: Imaging board
- 19c: Control board
- 19d: Wireless board
- 20: Series of circuit boards
- 20a, 20b: Series of flexible boards
- A1 to A5: Extending part
- CL: Central axis
- E1, E2: Upper end
- H1, H2: Opening part

### BEST MODE(S) FOR CARRYING OUT THE INVENTION

Exemplary embodiments of a capsule medical device and a method of manufacturing a capsule medical device according to the present invention will be explained below in detail with reference to the accompanying drawings. A capsule endoscope introduced into a subject and having an imaging function for capturing an in-vivo image, which is an example of in-vivo information of the subject, and a wireless communication function for wirelessly transmitting the captured in-vivo image is explained as an example of the capsule medical device of the present invention. However, the present invention is not limited to the embodiments.

### (First embodiment)

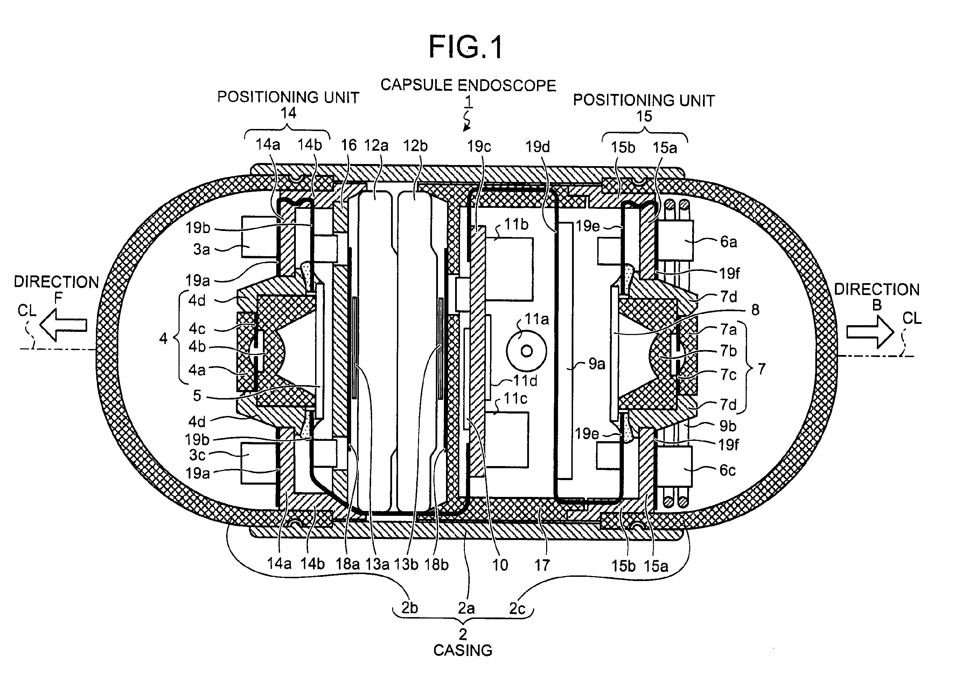

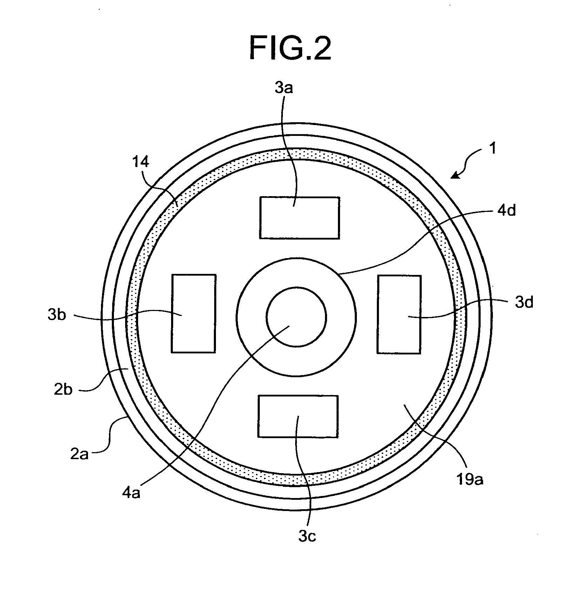

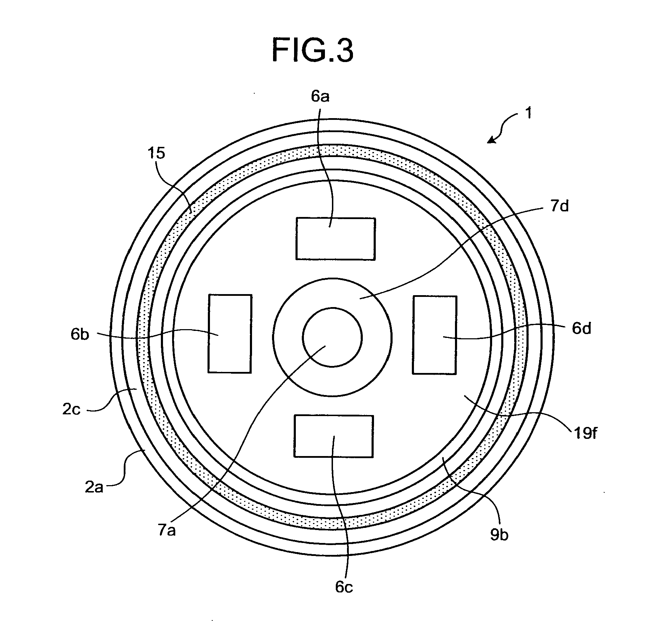

FIG. 1 is a longitudinal cross section of a configuration example of a capsule endoscope according to a first embodiment of the present invention. FIG. 2 is a schematic diagram for exemplifying an internal structure of the capsule endoscope as viewed over an optical dome from a direction F shown in FIG. 1. FIG. 3 is a schematic diagram for exemplifying the internal structure of the capsule endoscope as viewed over the optical dome from a direction B shown in FIG. 1.

As shown in FIG. 1, a capsule endoscope 1 according to the embodiment of the present invention is a binocular-lens capsule endoscope that captures an in-vivo image on a direction F side (forward side) and an in-vivo image on a direction B side (back side). The capsule endoscope 1 includes a capsule casing 2 formed in a size introduceable into internal organs of a subject, and has an imaging function for capturing an in-vivo image on the direction F side, an imaging function for capturing an in-vivo image on the direction B side, and a wireless communication function for wirelessly transmitting in-vivo images captured by these imaging functions to the outside.

Specifically, as shown in FIGS. 1 to 3, the capsule endoscope 1 includes, in the casing 2, an illuminating board 19a including a plurality of light-emitting elements 3a to 3d mounted thereon to illuminate the inside of the subject on the direction F side; an optical unit 4 that forms images of inside the subject illuminated by the light-emitting elements 3a to 3d; and an imaging board 19b including a solid-state imaging device 5 mounted thereon to capture the images of inside the subject formed by the optical unit 4 (that is, the in-vivo image on the direction F side). The capsule endoscope 1 also includes, in the casing 2, an illuminating board 19f including a plurality of light-emitting elements 6a to 6d mounted thereon to illuminate the inside of the subject on the direction B side; an optical unit 7 that forms images of inside the subject illuminated by the light-emitting elements 6a to 6d; and an imaging board 19e including a solid-state imaging device 8 mounted thereon to capture the images of inside the subject formed by the optical unit 7 (that is, the in-vivo image on the direction B side). Further, the capsule endoscope 1 includes, in the casing 2, a wireless board 19d having a wireless unit 9a mounted thereon to wirelessly transmit respective in-vivo images captured by the solid-state imaging devices 5 and 8 to the outside via an antenna 9b, and a control board 19c having a control unit 10 mounted thereon to control the imaging function and the wireless communication function.

The capsule endoscope 1 includes, in the casing 2, a power supply system for supplying electric power to the light-emitting elements 3a to 3d and 6a to 6d, the solid-state imaging devices 5 and 8, the wireless unit 9a, and the control unit 10, that is, various circuit parts such as a magnetic switch 11a; batteries 12a and 12b; power supply boards 18a and 18b; and contact springs 13a and 13b that connect the batteries 12a and 12b with the power supply boards 18a and 18b so that electrical conduction therebetween is established. Further, the capsule endoscope 1 also includes, in the casing 2, a positioning unit 14 that determines respective relative positions of the light-emitting elements 3a to 3d and the optical unit 4 with respect to an optical dome 2b forming the forward end of the casing 2; a positioning unit 15 that determines respective relative positions of the light-emitting elements 6a to 6d and the optical unit 7 with respect to an optical dome 2c forming the backward end of the casing 2; a load receiving unit 16 that receives an elastic force of the contact spring 13a to fix the positioning unit 14 with respect to the optical dome 2b; and a load receiving unit 17 that receives an elastic force of the contact spring 13b to fix the positioning unit 15 with respect to the optical dome 2c.

The casing 2 is a capsule casing having a size easily introduceable into the internal organs of the subject, and is realized by fitting the optical domes 2b and 2c to both opening end portions of a cylindrical body 2a having a cylindrical structure. The cylindrical body 2a has an outer diameter larger than that of the optical domes 2b and 2c, so that the optical domes 2b and 2c can be fitted to an inner circumference near the both opening ends. A step that abuts against the end face of the optical domes 2b and 2c at the time of fitting the optical domes 2b and 2c is formed on the inner circumference near the both opening ends of the cylindrical body 2a. The relative positions of the optical domes 2b and 2c with respect to the cylindrical body 2a are determined by abutting the respective end faces of the optical domes 2b and 2c against the step of the cylindrical body 2a.

The optical domes 2b and 2c are optically transparent dome members formed in a substantially uniform thickness. A depression is formed on an outer circumference near the opening end of each of the optical domes 2b and 2c. The depressions engage with protrusions provided on the inner circumference near the opening ends of the cylindrical body 2a. The optical dome 2b is fitted to the inner circumference near the opening end on the forward side (the direction F side shown in FIG. 1) of the cylindrical body 2a, and is attached to the forward-side opening end of the cylindrical body 2a by locking the protrusion on the inner circumference of the cylindrical body 2a in the depression of the optical dome 2b. In this case, the end face of the optical dome 2b is in a state of being abutted against the step on the inner circumference of the cylindrical body 2a. The optical dome 2b forms a part of the capsule casing 2 (specifically, a forward end). Meanwhile, the optical domes 2c is fitted to the inner circumference near the opening end on the back side (the direction B side shown in FIG. 1) of the cylindrical body 2a, and is attached to the back-side opening end of the cylindrical body 2a by locking the protrusion on the inner circumference of the cylindrical body 2a in the depression of the optical dome 2c. In this case, the end face of the optical dome 2c is in a state of being abutted against the step on the inner circumference of the cylindrical body 2a. The optical dome 2c forms a part of the capsule casing 2 (specifically, a backward end). As shown in FIG. 1, the casing 2 including the cylindrical body 2a and the optical domes 2b and 2c liquid-tightly accommodates the respective components of the capsule endoscope 1.

The light-emitting elements 3a to 3d function as an illuminating unit that illuminates the inside of the subject positioned on the direction F side. Specifically, each of the light-emitting elements 3a to 3d is a light-emitting element such as an LED, and is mounted on the illuminating board 19a, which is a flexible board formed in a substantially disk shape. In this case, as shown in FIGS. 1 and 2, the light-emitting elements 3a to 3d are mounted on the illuminating board 19a to surround a lens frame 4d (described later) of the optical unit 4 inserted into an opening part of the illuminating board 19a. The light-emitting elements 3a to 3d emit predetermined illumination light (for example, white light), to illuminate the inside of the subject on the direction F side over the forward-side optical dome 2b.

The number of the light-emitting elements to be mounted on the illuminating board 19a is not specifically limited to four, and can be one or more, so long as the light-emitting element can emit the illumination light with an amount of light sufficient for illuminating the inside of the subject on the direction F side. As exemplified in the light-emitting elements 3a to 3d, when a plurality of light-emitting elements are mounted on the illuminating board 19a, it is desired that the light-emitting elements are mounted thereon at rotationally symmetric positions centering on an optical axis of the optical unit 4 inserted into the opening part of the illuminating board 19a.

The optical unit 4 condenses reflected light from the inside of the subject on the direction F side illuminated by the light-emitting elements 3a to 3d, and forms images of inside the subject on the direction F side. The optical unit 4 is realized by lenses 4a and 4b formed by, for example, injection molding of glass or plastic, an aperture unit 4c arranged between the lenses 4a and 4b, and the lens frame 4d that holds the lenses 4a and 4b and the aperture unit 4c.

The lenses 4a and 4b condense the reflected light from the inside of the subject on the direction F side illuminated by the light-emitting elements 3a to 3d, and forms images of inside the subject on the direction F side on a light receiving surface of the solid-state imaging device 5. The aperture unit 4c narrows down (adjusts) brightness of the reflected light condensed by the lenses 4a and 4b to suitable brightness. The lens frame 4d has a cylindrical structure with the both ends being opened, and holds the lenses 4a and 4b and the aperture unit 4c in a cylindrical portion. The lens frame 4d is fitted and fixed to a through hole in a plate-like portion 14a (described later) of the positioning unit 14, with the lens frame 4d being inserted into an opening part formed in the illuminating board 19a. In this case, an upper end (an opening end on the lens 4a side) and a body of the lens frame 4d are protruded on the illuminating board 19a side, and a lower end thereof is locked to a peripheral portion of the through hole in the plate-like portion 14a. The lens frame 4d fixed to the plate-like portion 14a of the positioning unit 14 holds the lenses 4a and 4b at predetermined positions determined by the positioning unit 14 (that is, suitable relative positions with respect to the optical dome 2b). The lenses 4a and 4b can match a longitudinal central axis CL of the casing 2 with the optical axis.

The lens 4b held by the lens frame 4d has legs as shown in FIG. 1, and determines positional relation between the lens 4b and the solid-state imaging device 5 in an optical axis direction by abutting the legs against a device surface on a light receiving side of the solid-state imaging device 5. Thus, in a manner in which the legs of the lens 4b abut against the device surface on the light receiving side of the solid-state imaging device 5, a clearance is formed between the lower end of the lens frame 4d and the imaging board 19b. A predetermined adhesive is filled in the clearance, and the lower end of the lens frame 4d and the imaging board 19b are bonded to each other by the adhesive. The adhesive and the lens frame 4d block unnecessary light from entering into the lenses 4a and 4b and the light receiving surface of the solid-state imaging device 5.

The solid-state imaging device 5 is a CCD, CMOS, or the like having the light receiving surface, and functions as an imaging unit that captures images of inside the subject on the direction F side illuminated by the light-emitting elements 3a to 3d. Specifically, the solid-state imaging device 5 is mounted (for example, flip-chip mounted) on the imaging board 19b, which is the flexible board formed in a substantially disk shape, so that the lens 4b faces the light receiving surface via an opening part of the imaging board 19b. In this case, the solid-state imaging device 5 causes the device surface thereof on the light receiving side to abut against the legs of the lens 4b, and is fixed and arranged with respect to the optical unit 4 by adhesion between the imaging board 19b and the lower end of the lens frame 4d, while maintaining the abutting state with respect to the legs of the lens 4b. The solid-state imaging device 5 receives the reflected light from the inside of the subject condensed by the lenses 4a and 4b via the light receiving surface, and captures images of inside the subject formed on the light receiving surface by the lenses 4a and 4b (that is, an in-vivo image on the direction F side).

The light-emitting elements 6a to 6d function as an illuminating unit that illuminates the inside of the subject positioned on the direction B side. Specifically, each of the light-emitting elements 6a to 6d is a light-emitting element such as an LED, and is mounted on the illuminating board 19f, which is a flexible board formed in a substantially disk shape. In this case, as shown in FIGS. 1 and 3, the light-emitting elements 6a to 6d are mounted on the illuminating board 19f to surround a lens frame 7d (described later) of the optical unit 7 inserted into an opening part of the illuminating board 19f. The light-emitting elements 6a to 6d emit predetermined illumination light (for example, white light), to illuminate the inside of the subject on the direction B side over the back-side optical dome 2c.

The number of the light-emitting elements to be mounted on the illuminating board 19f is not specifically limited to four, and can be one or more, so long as the light-emitting element can emit the illumination light with an amount of light sufficient for illuminating the inside of the subject on the direction B side. As exemplified in the light-emitting elements 6a to 6d, when a plurality of light-emitting elements are mounted on the illuminating board 19f, it is desired that the light-emitting elements are mounted thereon at rotationally symmetric positions centering on an optical axis of the optical unit 7 inserted into the opening part of the illuminating board 19f.

The optical unit 7 condenses the reflected light from the inside of the subject on the direction B side illuminated by the light-emitting elements 6a to 6d and forms images of inside the subject on the direction B side. The optical unit 7 is realized by lenses 7a and 7b formed by, for example, injection molding of glass or plastic, an aperture unit 7c arranged between the lenses 7a and 7b, and the lens frame 7d that holds the lenses 7a and 7b and the aperture unit 7c.

The lenses 7a and 7b condense the reflected light from the inside of the subject on the direction B side illuminated by the light-emitting elements 6a to 6d, and forms the images of inside the subject on the direction B side on a light receiving surface of the solid-state imaging device 8. The aperture unit 7c narrows down (adjusts) brightness of the reflected light condensed by the lenses 7a and 7b to suitable brightness. The lens frame 7d has a cylindrical structure with the both ends being opened, and holds the lenses 7a and 7b and the aperture unit 7c in a cylindrical portion. The lens frame 7d is fitted and fixed to a through hole in a plate-like portion 15a (described later) of the positioning unit 15, with the lens frame 7d being inserted into the opening part formed in the illuminating board 19f. In this case, an upper end (an opening end on the lens 7a side) and a body of the lens frame 7d are protruded on the illuminating board 19f side, and a lower end thereof is locked to a peripheral portion of the through hole in the plate-like portion 15a. The lens frame 7d fixed to the plate-like portion 15a of the positioning unit 15 holds the lenses 7a and 7b at predetermined positions determined by the positioning unit 15 (that is, suitable relative positions with respect to the optical dome 2c). The lenses 7a and 7b can match the longitudinal central axis CL of the casing 2 with the optical axis.

The lens 7b held by the lens frame 7d has legs (see FIG. 1) as the lens 4b of the optical unit 4, and determines positional relation between the lens 7b and the solid-state imaging device 8 in the optical axis direction by abutting the legs against a device surface on a light receiving side of the solid-state imaging device 8. Thus, in a manner in which the legs of the lens 7b abut against the device surface on the light receiving side of the solid-state imaging device 8, a clearance is formed between the lower end of the lens frame 7d and the imaging board 19e. A predetermined adhesive is filled in the clearance, and the lower end of the lens frame 7d and the imaging board 19e are bonded to each other by the adhesive. The adhesive and the lens frame 7d block unnecessary light from entering into the lenses 7a and 7b and the light receiving surface of the solid-state imaging device 8.

The solid-state imaging device 8 is a CCD, CMOS, or the like having the light receiving surface, and functions as an imaging unit that captures images of inside the subject on the direction B side illuminated by the light-emitting elements 6a to 6d. Specifically, the solid-state imaging device 8 is mounted (for example, flip-chip mounted) on the imaging board 19e, which is a flexible board formed in a substantially disk shape, so that the lens 7b faces the light receiving surface via an opening part of the imaging board 19e. In this case, the solid-state imaging device 8 causes the device surface thereof on the light receiving side to abut against the legs of the lens 7b, and is fixed and arranged with respect to the optical unit 7 by adhesion between the imaging board 19e and the lower end of the lens frame 7d, while maintaining the abutting state with respect to the legs of the lens 7b. The solid-state imaging device 8 receives the reflected light from the inside of the subject condensed by the lenses 7a and 7b via the light receiving surface, and captures images of inside the subject formed on the light receiving surface by the lenses 7a and 7b (that is, an in-vivo image on the direction B side).

The wireless unit 9a and the antenna 9b realize the wireless communication function for wirelessly transmitting each of in-vivo images on the direction F or the direction B side captured by the solid-state imaging devices 5 and 8 to the outside. Specifically, the wireless unit 9a is mounted on the wireless board 19d, which is the flexible board formed in a substantially disk shape, and is arranged in the casing 2, facing the imaging board 19e having the solid-state imaging device 8 mounted thereon. As shown in FIGS. 1 and 3, the antenna 9b is fixed and arranged on the illuminating board 19f fixed on the surface of the plate-like portion 15a of the positioning unit 15, and is connected to the wireless unit 9a via the wireless board 19d and the illuminating board 19f. In this case, the antenna 9b is fixed and arranged on an outer edge of the illuminating board 19f facing the optical dome 2c at the backward end and outside of the light-emitting elements 6a to 6d.

When having acquired an image signal including the in-vivo image on the direction F side captured by the solid-state imaging device 5, the wireless unit 9a performs modulation or the like with respect to the acquired image signal each time, to generate a wireless signal including the in-vivo image on the direction F side, and transmits the generated wireless signal to the outside via the antenna 9b. Meanwhile, when having acquired an image signal including the in-vivo image on the direction B side captured by the solid-state imaging device 8, the wireless unit 9a performs modulation or the like with respect to the acquired image signal each time, to generate a wireless signal including the in-vivo image on the direction B side, and transmits the generated wireless signal to the outside via the antenna 9b. The wireless unit 9a alternately generates the wireless signal including the in-vivo image on the direction F side and the wireless signal including the in-vivo image on the direction B side under control of the control unit 10, and alternately transmits the generated wireless signals.

The control unit 10 is a processor such as a DSP, and is arranged approximately at the center of the casing 2 in a state mounted on the control board 19c, which is a rigid board formed in a substantially disk shape. The control unit 10 is electrically connected to the illuminating boards 19a and 19f, the imaging boards 19b and 19e, and the wireless board 19d via the control board 19c and the flexible board. The control unit 10 controls: the light-emitting elements 3a to 3d mounted on the illuminating board 19a; the light-emitting elements 6a to 6d mounted on the illuminating board 19f; the solid-state imaging devices 5 and 8 mounted on the imaging boards 19b and 19e, respectively; and the wireless unit 9a mounted on the wireless board 19d. Specifically, the control unit 10 controls operation timing of the light-emitting elements 3a to 3d and the solid-state imaging device 5 so that the solid-state imaging device 5 captures the in-vivo image on the direction F side for each predetermined time period, synchronously with a light emitting operation of the light-emitting elements 3a to 3d. Likewise, the control unit 10 controls the operation timing of the light-emitting elements 6a to 6d and the solid-state imaging device 8 so that the solid-state imaging device 8 captures the in-vivo image on the direction B side for each predetermined time period, synchronously with the light emitting operation of the light-emitting elements 6a to 6d. The control unit 10 also controls the wireless unit 9a to wirelessly transmit the in-vivo image on the direction F side and the in-vivo image on the direction B side alternately. The control unit 10 includes various parameters involved with image processing such as white balance, and has an image processing function for sequentially generating the image signal including the in-vivo image on the direction F side captured by the solid-state imaging device 5 and the image signal including the in-vivo image on the direction B side captured by the solid-state imaging device 8.

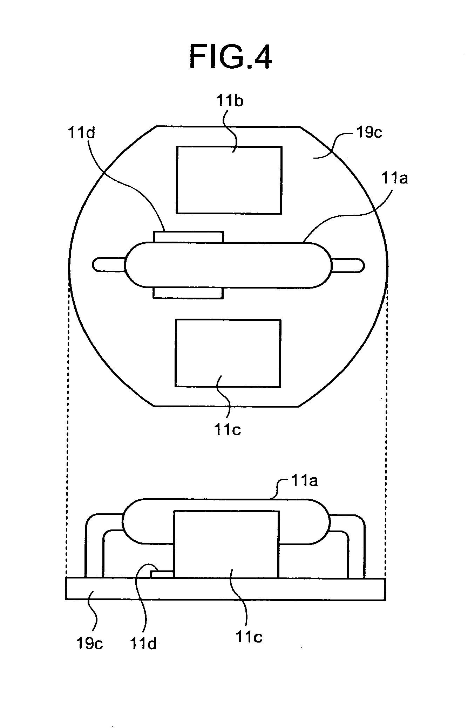

Meanwhile, on the control board 19c, circuit components of the power supply system, that is, various circuit components such as the magnetic switch 11a are mounted on a board surface on the opposite side of the board surface where the control unit 10 is mounted. FIG. 4 is a schematic diagram for exemplifying a state where the circuit components of the power supply system are mounted on the control board 19c. As shown in FIGS. 1 and 4, for example, the magnetic switch 11a, capacitors 11b and 11c, and a power supply IC 11d are mounted on one board surface of the control board 19c, as the circuit components of the power supply system. In this case, the capacitors 11b and 11c and the power supply IC 11d are surface-mounted on the control board 19c, and the magnetic switch 11a is mounted on the control board 19c, spanning over the power supply IC 11d using a lead extending from the both ends of the magnetic switch 11a. The magnetic switch 11a switches ON/OFF by applying an external magnetic field in a predetermined direction. In a case of ON state, the magnetic switch 11a starts to supply power to the light-emitting elements 3a to 3d and 6a to 6d, the solid-state imaging devices 5 and 8, the wireless unit 9a, and the control unit 10 from the batteries 12a and 12b, and in a case of OFF state, the magnetic switch 11a stops supplying power from the batteries 12a and 12b. Meanwhile, the power supply IC 11d has a power supply control function for controlling the power supply to the respective components via the magnetic switch 11a.

The batteries 12a and 12b generate power for operating the light-emitting elements 3a to 3d and 6a to 6d, the solid-state imaging devices 5 and 8, the wireless unit 9a, and the control unit 10. Specifically, the batteries 12a and 12b are button batteries such as a silver oxide battery, and as shown in FIG. 1, are arranged between the load receiving units 16 and 17 and held by an end of the positioning unit 14 and an end of the load receiving unit 17. The power supply boards 18a and 18b electrically connected to the control board 19c via the flexible board or the like are provided on surfaces of the load receiving units 16 and 17, respectively, which are facing the batteries 12a and 12b, respectively. The conductive contact springs 13a and 13b are provided on the power supply boards 18a and 18b, respectively. The batteries 12a and 12b arranged between the load receiving units 16 and 17 are held by the end of the positioning unit 14 and the end of the load receiving unit 17 in a manner in which the contact springs 13a and 13b are contracted, and are electrically connected to the circuit components (the magnetic switch 11a, the capacitors 11b and 11c, and the power supply IC 11d) of the power supply system on the control board 19c via the contracted contact springs 13a and 13b and the power supply boards 18a and 18b. The number of batteries arranged in the casing 2 is not particularly limited two, so long as the required power can be supplied.

The illuminating board 19a including the light-emitting elements 3a to 3d mounted thereon and the optical unit 4 are fixed and arranged in the positioning unit 14, and the positioning unit 14 is fitted and fixed to an inner circumference of the forward-side optical dome 2b. The positioning unit 14 fitted and fixed to the inner circumference of the optical dome 2b fixes the positional relation of the optical dome 2b, the light-emitting elements 3a to 3d, and the optical unit 4, and determines suitable relative positions of the light-emitting elements 3a to 3d and the optical unit 4 with respect to the optical dome 2b. The positioning unit 14 includes the plate-like portion 14a fitted to the inner circumference of the optical dome 2b and a protrusion 14b for fixing the plate-like portion 14a at a predetermined position on the inner circumference of the optical dome 2b.

The plate-like portion 14a is a substantially disk plate member having an outer diameter matched with an inner diameter of the optical dome 2b, and has an outer circumference fitted to the inner circumference of the optical dome 2b. The illuminating board 19a and the optical unit 4 are fixed and arranged on the plate-like portion 14a. Specifically, the plate-like portion 14a fixes and arranges the illuminating board 19a on a surface facing the optical dome 2b, when being fitted to the inner circumference of the optical dome 2b. The plate-like portion 14a has a through hole that communicates with an opening part formed in the illuminating board 19a substantially at a center thereof, and the lens frame 4d of the optical unit 4 is inserted into and fixed (for example, fitted and fixed) in the through hole. The lens frame 4d inserted into and fixed in the through hole of the plate-like portion 14a protrudes the upper end and the body thereof on the illuminating board 19a side in a state of being inserted into the opening part of the illuminating board 19a. The plate-like portion 14a fixes the positional relation between the lens frame 4d and the light-emitting elements 3a to 3d so that the respective upper ends of the light-emitting elements 3a to 3d are positioned at a lower position than the upper end of the lens frame 4d.

The protrusion 14b protrudes from the plate-like portion 14a, and is locked to the opening end of the optical dome 2b to fix the plate-like portion 14a on the inner circumference of the optical dome 2b. Specifically, the protrusion 14b is integrally formed with the plate-like portion 14a, and protrudes from a back of the surface of the plate-like portion 14a, on which the illuminating board 19a is fixed and arranged. The protrusion 14b has a cylindrical structure having an outer diameter matched with the inner diameter of the optical dome 2b (that is, outer diameter same as that of the plate-like portion 14a), and includes a flange that engages with the opening end of the optical dome 2b at the opening end of the cylindrical structure. The protrusion 14b having such a structure is fitted to the inner circumference of the optical dome 2b together with the plate-like portion 14a, and locks the flange to the opening end of the optical dome 2b. Accordingly, the protrusion 14b fixes the plate-like portion 14a at the predetermined position on the inner circumference of the optical dome 2b.

The illuminating board 19f including the light-emitting elements 6a to 6d mounted thereon and the optical unit 4 are fixed and arranged in the positioning unit 15, and the positioning unit 15 is fitted and fixed to an inner circumference of the backward-side optical dome 2c. The positioning unit 15 fitted and fixed to the inner circumference of the optical dome 2c fixes the positional relation of the optical dome 2c, the light-emitting elements 6a to 6d, and the optical unit 7, and determines suitable relative positions of the light-emitting elements 6a to 6d and the optical unit 7 with respect to the optical dome 2c. The positioning unit 15 includes the plate-like portion 15a fitted to the inner circumference of the optical dome 2c and a protrusion 15b for fixing the plate-like portion 15a at a predetermined position on the inner circumference of the optical dome 2c.

The plate-like portion 15a is a substantially disk plate member having an outer diameter matched with an inner diameter of the optical dome 2c, and has an outer circumference fitted to the inner circumference of the optical dome 2c. The illuminating board 19f and the optical unit 7 are fixed and arranged on the plate-like portion 15a. Specifically, the plate-like portion 15a fixes and arranges the illuminating board 19f on a surface facing the optical dome 2c, when being fitted to the inner circumference of the optical dome 2c. The plate-like portion 15a has a through hole that communicates with an opening part formed in the illuminating board 19f substantially at a center thereof, and the lens frame 7d of the optical unit 7 is inserted into and fixed (for example, fitted and fixed) in the through hole. The lens frame 7d inserted into and fixed in the through hole of the plate-like portion 15a protrudes the upper end and the body thereof on the illuminating board 19f side in a state of being inserted into the opening part of the illuminating board 19f. The plate-like portion 15a fixes the positional relation between the lens frame 7d and the light-emitting elements 6a to 6d so that the respective upper ends of the light-emitting elements 6a to 6d are positioned at a lower position than the upper end of the lens frame 7d.

The protrusion 15b protrudes from the plate-like portion 15a, and is locked to the opening end of the optical dome 2c to fix the plate-like portion 15a on the inner circumference of the optical dome 2c. Specifically, the protrusion 15b is integrally formed with the plate-like portion 15a, and protrudes from a back of the surface of the plate-like portion 15a, on which the illuminating board 19f is fixed and arranged. The protrusion 15b has a cylindrical structure having an outer diameter matched with the inner diameter of the optical dome 2c (that is, outer diameter same as that of the plate-like portion 15a), and includes a flange that engages with the opening end of the optical dome 2c at the opening end of the cylindrical structure. The protrusion 15b having such a structure is fitted to the inner circumference of the optical dome 2c together with the plate-like portion 15a, and locks the flange to the opening end of the optical dome 2c. Accordingly, the protrusion 15b fixes the plate-like portion 15a at the predetermined position on the inner circumference of the optical dome 2c.

Upon reception of the elastic force (spring force) of the contact spring 13a, the load receiving unit 16 presses and fixes the positioning unit 15 to the opening end of the optical dome 2c by the elastic force. Specifically, the load receiving unit 16 is a plate member having a substantially disk shape that engages the outer edge thereof with a step formed on an inner circumference of the protrusion 15b of the positioning unit 14, and includes the power supply board 18a and the contact spring 13a on the surface facing the battery 12a. The load receiving unit 16 presses and fixes the flange of the protrusion 14b to the opening end of the optical dome 2b by the elastic force of the contact spring 13a, upon reception of the elastic force of the contact spring 13a generated with contraction of the contact spring 13a. In this case, the load receiving unit 16 fits and fixes the plate-like portion 14a integral with the protrusion 14b at the predetermined position on the inner circumference of the optical dome 2b by pressing and fixing the protrusion 14b to the opening end of the optical dome 2b.

As shown in FIG. 1, the through hole for avoiding a contact with the circuit components such as the capacitor mounted on the imaging board 19b is provided in the load receiving unit 16. When the load receiving unit 16 is engaged with the step on the inner circumference of the protrusion 14b, the load receiving unit 16 and the positioning unit 14 form a space, as shown in FIG. 1, sufficient for arranging the solid-state imaging device 5 abutting against the legs of the lens 4b and the imaging board 19b fixed with respect to the lower part of the lens frame 4d.

Upon reception of the elastic force (spring force) of the contact spring 13b, the load receiving unit 17 presses and fixes the positioning unit 15 to the opening end of the optical dome 2c by the elastic force. Specifically, the load receiving unit 17 is a member having a cylindrical structure having a slightly smaller outer diameter than an inner diameter of the cylindrical body 2a of the casing 2, and including a plate-like portion facing the battery 12b at one opening end of the cylindrical structure.

The cylindrical structure of the load receiving unit 17 functions as a spacer that forms a predetermined space in the casing 2, and engages the other opening end with the opening end (flange) of the protrusion 15b of the positioning unit 15. In this case, as shown in FIG. 1, the cylindrical structure of the load receiving unit 17 and the positioning unit 15 forms a space sufficient for arranging the control board 19c including the control unit 10 and the circuit components such as the magnetic switch 11a mounted thereon, the wireless board 19d including the wireless unit 9a mounted thereon, the solid-state imaging device 8 abutting against the legs of the lens 7b, and the imaging board 19e fixed with respect to the lower part of the lens frame 7d.

Meanwhile, the plate-like portion of the load receiving unit 17 is integrally formed with the cylindrical structure of the load receiving unit 17 at one opening end thereof, and as shown in FIG. 1, includes the power supply board 18b and the contact spring 13b on the surface facing the battery 12b. The plate-like portion of the load receiving unit 17 has a through hole for preventing a contact with the circuit components such as the capacitor mounted on the control board 19c, arranged in the space formed by the cylindrical structure of the load receiving unit 17. The plate-like portion of the load receiving unit 17 receives the elastic force of the contact spring 13b generated with contraction of the contact spring 13b, and presses the cylindrical structure of the load receiving unit 17 to the opening end of the protrusion 15b of the positioning unit 15 by the elastic force of the contact spring 13b.

The load receiving unit 17 having the cylindrical structure and the plate-like portion presses and fixes the flange of the protrusion 15b to the opening end of the optical dome 2c by the elastic force of the contact spring 13b. In this case, the load receiving unit 17 presses and fixes the protrusion 15b to the opening end of the optical dome 2c, thereby fitting and fixing the plate-like portion 15a integral with the protrusion 15b to a predetermined position on the inner circumference of the optical dome 2c.

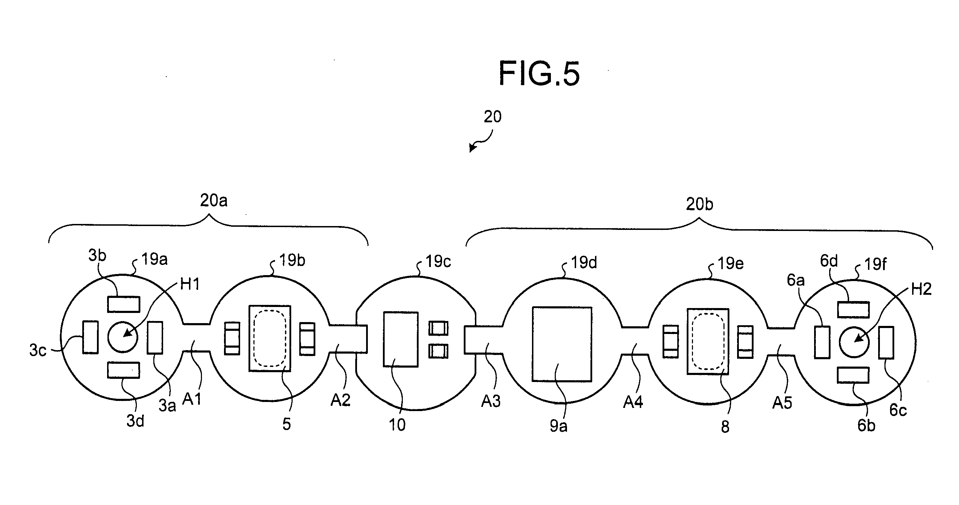

A series of circuit boards (specifically, the illuminating boards 19a and 19f, the imaging boards 19b and 19e, the control board 19c, and the wireless board 19d) arranged in the casing 2 of the capsule endoscope 1 is explained next. FIG. 5 is a schematic diagram for exemplifying a state where the series of circuit boards folded and arranged in the casing 2 of the capsule endoscope 1 is developed. Each board surface of the flexible board or the rigid board shown in FIG. 5 is defined as a board surface at the front (front board surface), and a back face of the front board surface shown in FIG. 5 is defined as a board surface at the back (back board surface).

As shown in FIG. 5, a series of circuit boards 20 arranged in the casing 2 of the capsule endoscope 1 is achieved by electrically connecting a series of flexible boards 20a connecting the illuminating board 19a and the imaging board 19b, the control board 19c as the rigid board, and a series of flexible boards 20b connecting the wireless board 19d, the imaging board 19e, and the illuminating board 19f.

The illuminating board 19a is flexible board having a substantially disk shape, on which a circuit for realizing an illuminating function for illuminating the subject on the direction F side of the capsule endoscope 1 is formed. The plurality of light-emitting elements 3a to 3d are mounted on the front board surface of the illuminating board 19a, and an opening part H1 for inserting the lens frame 4d of the optical unit 4 having the lens 4b, in a manner in which the legs thereof abut against the solid-state imaging device 5, is formed at the center of the board surface of the illuminating board 19a surrounded by the light-emitting elements 3a to 3d. The illuminating board 19a is electrically connected to the imaging board 19b via an extending part A1, which is a flexible board extending from an outer edge.

The imaging board 19b is a flexible board having a substantially disk shape, on which a circuit for realizing the imaging function for capturing the in-vivo image on the direction F side is formed. The solid-state imaging device 5 is flip-chip mounted on the front board surface of the imaging board 19b, and the circuit components such as the capacitor are mounted thereon as required. As shown by a dotted line in FIG. 5, in the imaging board 19b, there is formed an opening part for the reflected light from inside of the subject on the direction F side to enter into a light-receiving surface of the flip-chip mounted solid-state imaging device 5. Although not specifically shown in FIG. 5, the lower end of the lens frame 4d of the optical unit 4 abutting against the legs of the lens 4b is fixed on the light-receiving side device surface of the solid-state imaging device 5 via the opening part of the imaging board 19b, as shown in FIG. 1. The imaging board 19b is electrically connected to the control board 19c via an extending part A2, which is a flexible board extending from the outer edge.

The control board 19c is a rigid board having a substantially disk shape, on which a circuit necessary for the power supply system such as the magnetic switch 11a and the control unit 10 is formed. The control unit 10 is mounted on the front board surface of the control board 19c, and the circuit components such as the capacitor are mounted thereon as required. Meanwhile, as shown in FIG. 4, the magnetic switch 11a, the capacitors 11b and 11c, and the power supply IC 11d, which are the circuit components of the power supply system, are mounted on the back board surface of the control board 19c. The control board 19c is electrically connected to the wireless board 19d via an extending part A3, which is a flexible board extending from the outer edge of the wireless board 19d. Although not specifically shown in FIG. 5, the control board 19c is electrically connected to the power supply boards 18a and 18b via the flexible board or the like (not shown).

The wireless board 19d is a flexible board having a substantially disk shape, on which a circuit for realizing the wireless communication function for wirelessly transmitting the in-vivo image on the direction F side and the in-vivo image on the direction B side sequentially to the outside is formed. The wireless unit 9a is mounted on the front board surface of the wireless board 19d. Although not particularly shown in FIG. 5, the wireless board 19d is electrically connected to the antenna 9b fixed and arranged on the outer edge of the illuminating board 19f, as shown in FIGS. 1 and 3. The wireless board 19d is electrically connected to the imaging board 19e via an extending part A4, which is a flexible board extending from the outer edge.

The imaging board 19e is a flexible board having a substantially disk shape, on which a circuit for realizing the imaging function for capturing the in-vivo image on the direction B side is formed. The solid-state imaging device 8 is flip-chip mounted on the front board surface of the imaging board 19e, and the circuit components such as the capacitor are mounted as required. As shown by a dotted line in FIG. 5, in the imaging board 19e, there is formed an opening part for the reflected light from inside of the subject on the direction F side to enter into a light-receiving surface of the flip-chip mounted solid-state imaging device 8. Although not specifically shown in FIG. 5, the lower end of the lens frame 7d of the optical unit 7 abutting against the legs of the lens 7b is fixed on the light-receiving side device surface of the solid-state imaging device 8 via the opening part of the imaging board 19e, as shown in FIG. 1. The imaging board 19e is electrically connected to the illuminating board 19f via an extending part A5, which is a flexible board extending from the outer edge.

The illuminating board 19f is a flexible board having a substantially disk shape, on which a circuit that realizes the illuminating function for illuminating the subject on the direction B side of the capsule endoscope 1 is formed. The light-emitting elements 6a to 6d described above are mounted on the front board surface of the illuminating board 19f, and an opening part H2 for inserting the lens frame 7d of the optical unit 7 having the lens 7b in a manner in which the legs abut against the solid-state imaging device 8 is formed at the center of the board surface of the illuminating board 19f surrounded by the light-emitting elements 6a to 6d.

The series of flexible board 20a is an integrally formed flexible board including the illuminating board 19a and the imaging board 19b, and has a board structure in which the imaging board 19b having the extending part A2 for connecting to the control board 19c extending from the outer edge thereof is connected to the illuminating board 19a via the extending part A1. On the other hand, the series of flexible board 20b is an integrally formed flexible board including the wireless board 19d, the imaging board 19e, and the illuminating board 19f, and has a board structure in which the wireless board 19d having the extending part A3 for connecting to the control board 19c extending from the outer edge thereof is connected to the imaging board 19e via the extending part A4, and a board structure in which the imaging board 19e and the illuminating board 19f are connected to each other via the extending part A5. The series of circuit board 20 arranged in the casing 2 of the capsule endoscope 1 is realized by connecting the series of flexible boards 20a and 20b with the control board 19c via the extending parts A2 and A3.

A manufacturing method of the capsule endoscope 1 according to the embodiment of the present invention is explained next. The capsule endoscope 1 is manufactured by preparing the series of circuit boards 20 shown in FIG. 5, preparing a functional unit by combining the manufactured series of circuit boards 20, the positioning units 14 and 15, the load receiving units 16 and 17, and the batteries 12a and 12b, and arranging the manufactured functional unit in the casing 2.

Specifically, the necessary functional components are first mounted on the illuminating board 19a and the imaging board 19b in the series of flexible boards 20a, and the necessary functional components are then mounted on the wireless board 19d, the imaging board 19e, and the illuminating board 19f in the series of flexible boards 20b. In this case, in the series of flexible boards 20a, a plurality of light-emitting elements 3a to 3d are mounted on the front board surface of the illuminating board 19a, the solid-state imaging device 5 and the circuit components such as the capacitor are mounted on the front board surface of the imaging board 19b, and the optical unit 4 is mounted on the back board surface of the imaging board 19b in a manner in which the legs of the lens 4b abut against the solid-state imaging device 5. Further, in the series of flexible boards 20b, a plurality of light-emitting elements 6a to 6d and the antenna 9b are mounted on the front board surface of the illuminating board 19f, the solid-state imaging device 8 and the circuit components such as the capacitor are mounted on the front board surface of the imaging board 19e, and the optical unit 7 is mounted on the back board surface of the imaging board 19e in a manner in which the legs of the lens 7b abut against the solid-state imaging device 8. Meanwhile, the control unit 10 and the circuit components such as the capacitor are mounted on the front board surface of the control board 19c, and the circuit components of the power supply system such as the magnetic switch 11a are mounted on the back board surface of the control board 19c. The series of circuit boards 20 is manufactured by connecting the series of flexible boards 20a and 20b.

The lens frame 4d of the optical unit 4 is a separate body with respect to the positioning unit 14, and is mounted on the back board surface of the imaging board 19b before being fitted and fixed in a through hole of the positioning unit 14 (specifically, the plate-like portion 14a) as shown in FIG. 1. Therefore, a working space required for applying an adhesive to a clearance between the imaging board 19b and the lower end of the lens frame 4d can be ensured sufficiently, and the lens frame 4d can be easily fixed to the imaging board 19b by the adhesive. The same applies to the lens frame 7d fitted to the back board surface of the imaging board 19e.

The functional unit of the capsule endoscope 1 is then manufactured by combining the series of circuit boards 20 manufactured as described above, the positioning units 14 and 15, the load receiving units 16 and 17, and the batteries 12a and 12b. The functional unit is the one excluding the casing 2 of the capsule endoscope 1 shown in FIG. 1 (that is, a unit arranged in the casing 2).

In the functional unit, the lens frame 4d of the optical unit 4 mounted on the imaging board 19b is fitted and fixed in a through hole formed in the plate-like portion 14a of the positioning unit 14. An adhesive or a double-sided tape is applied or attached to one surface of the plate-like portion 14a (a surface facing the optical dome 2b) as a bonding member, and the illuminating board 19a is fixed to the plate-like portion 14a by the bonding member, with the lens frame 4d being inserted into the opening part H1. The outer edge of the load receiving unit 16 is engaged with the protrusion 14b of the positioning unit 14, to which the illuminating board 19a and the imaging board 19b are fitted. In this case, the load receiving unit 16 is fitted to the protrusion 14b in a manner in which the power supply board 18a and the contact spring 13a are arranged on the backward side of the surface facing the solid-state imaging device 5 of the imaging board 19b.

Meanwhile, the lens frame 7d of the optical unit 7 mounted on the imaging board 19e is fitted and fixed in the through hole formed in the plate-like portion 15a of the positioning unit 15. The adhesive or double-sided tape is applied or attached to one surface of the plate-like portion 15a (a surface facing the optical dome 2c) as a bonding member, and the illuminating board 19f is fixed to the plate-like portion 15a by the bonding member, with the lens frame 7d being inserted into the opening part H2. An end of the cylindrical structure of the load receiving unit 17 is engaged with the protrusion 15b of the positioning unit 15, to which the illuminating board 19f and the imaging board 19e are fitted. In this case, the load receiving unit 17 is fitted to the protrusion 15b in a state where the control board 19c and the wireless board 19d are arranged in the space formed by the cylindrical structure, and the power supply board 18b and the contact spring 13b can be arranged to face the power supply board 18a and the contact spring 13a of the load receiving unit 16.

Further, the batteries 12a and 12b are arranged between the load receiving units 16 and 17, in which the power supply board 18b and the contact spring 13b face the power supply board 18a and the contact spring 13a. In this case, the batteries 12a and 12b are held by the protrusion 14b of the positioning unit 14 an the end of the load receiving unit 17, with a positive pole and a negative pole thereof coming in contact with each other. The batteries 12a and 12b cause the contact springs 13a and 13b to contract, and are electrically connected to the power supply boards 18a and 18b via the contact springs 13a and 13b.

The functional unit of the capsule endoscope 1 is manufactured as described above. The series of circuit boards 20 incorporated in the functional unit is folded in a predetermined manner. In this case, as shown in FIG. 1, the back board surface of the illuminating board 19a and the back board surface of the imaging board 19b face each other via the plate-like portion 14a of the positioning unit 14, and the front board surface of the imaging board 19b and the front board surface of the control board 19c face each other via the load receiving units 16 and 17 and the batteries 12a and 12b. Further, the back board surface of the control board 19c and the back board surface of the wireless board 19d face each other, the front board surface of the wireless board 19d and the front board surface of the imaging board 19e face each other, and the back board surface of the imaging board 19e and the back board surface of the illuminating board 19f face each other via the plate-like portion 15a of the positioning unit 15. The extending part A1 is inserted into a notch (not shown) formed in the positioning unit 14, and the extending part A2 is inserted into notches (not shown) formed in the protrusion 14b of the positioning unit 14 and the load receiving unit 17. The extending part A3 is inserted into a notch (not shown) formed in the cylindrical structure of the load receiving unit 17, the extending part A4 is inserted into notches (not shown) formed in the opening end of the load receiving unit 17 and the protrusion 15b of the positioning unit 14, and the extending part A5 is inserted into a notch (not shown) formed in the positioning unit 15.

Thereafter, the functional unit described above is arranged in the capsule casing 2. That is, the functional unit is inserted into the cylindrical body 2a, and the optical domes 2b and 2c are fitted to respective inner circumferences near the both opening ends of the cylindrical body 2a, which houses the functional unit. In this case, as shown in FIG. 1, the optical domes 2b and 2c are fitted to the respective inner circumferences near the both opening ends of the cylindrical body 2a and fixed by the adhesive or the like, thereby completing the capsule endoscope 1 as shown in FIG. 1.

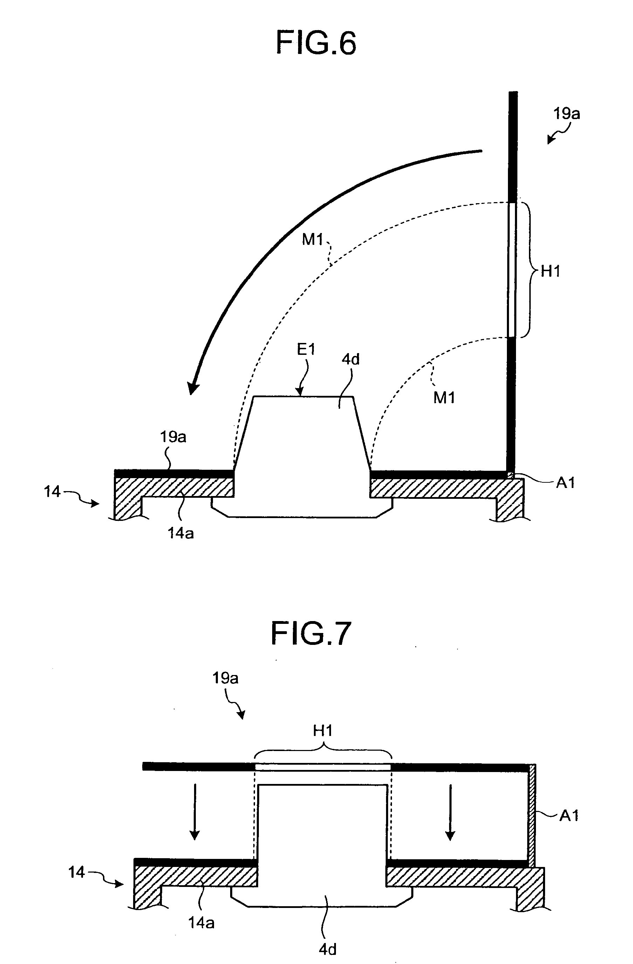

Fitting of the illuminating board 19a and the lens frame 4d is explained next. FIG. 6 is a schematic diagram for explaining fitting of the lens frame 4d fitted to the plate-like portion 14a of the positioning unit 14 and the illuminating board 19a.

As shown in FIGS. 1 and 6, the lens frame 4d of the optical unit 4 has a tapered external shape with one end (specifically, an upper end E1, which is an opening end on the lens frame 4a side) being tapered, and is fitted to the plate-like portion 14a of the positioning unit 14. In this case, the lens frame 4d having a tapered external shape is inserted into the through hole in the plate-like portion 14a from the tapered upper end E1. Accordingly, the outer circumference of the upper end E1 of the lens frame 4d and the through hole in the plate-like portion 14a can be easily aligned. As a result, the lens frame 4d can be easily fitted and fixed in the through hole in the plate-like portion 14a, with the upper end E1 of the lens frame 4d facing the optical dome 2b.

Thus, the illuminating board 19a is fitted to the lens frame 4d fitted and fixed to the plate-like portion 14a of the positioning unit 14. Specifically, as shown in FIG. 6, the illuminating board 19a is arranged on an upper face side (a surface facing the optical dome 2b shown in FIG. 1) of the plate-like portion 14a, continuously with the extending part A1 inserted into a notch (not shown) formed in the positioning unit 14. The extending part A1 is a flexible board extending to a part of the outer circumference (outer edge) of the illuminating board 19a, and functions as a bending part, which enables the illuminating board 19a to turn toward the upper face of the plate-like portion 14a by bending.

The illuminating board 19a having the extending part A1 on a part of the outer circumference turns around the extending part A1 in a direction of moving the opening part H1 toward the lens frame 4d, while bending the extending part A1. The illuminating board 19a is fixed to the upper face of the plate-like portion 14a while inserting the lens frame 4d into the opening part H1. Thus, by fixing the illuminating board 19a to the upper face of the plate-like portion 14a, with the lens frame 4d being inserted into the opening part H1, fitting of the lens frame 4d to the illuminating board 19a is complete.

Because the lens frame 4d has the tapered external shape at the upper end E1, a contact between the illuminating board 19a turning while bending the extending part A1 and the outer circumference of the upper end E1 can be avoided. In this case, as shown in FIG. 6, the upper end E1 of the lens frame 4d is positioned inside of a locus M1 drawn by a rim of the opening part H1 (that is, the inner circumference of the illuminating board 19a forming the opening part H1), when the illuminating board 19a is turned around the extending part A1 until it is surface-contacted with the plate-like portion 14a. The lens frame 4d having the tapered external shape facilitates alignment between the opening part H1 of the illuminating board 19a and the outer circumference of the upper end E1, and can be easily inserted into the opening part H1, while avoiding a contact between the illuminating board 19a turning while bending the extending part A1 and the outer circumference of the upper end E1.

On the other hand, if the lens frame 4d has a cylindrical shape and the cylindrical lens frame 4d is inserted into the opening part H1 of the illuminating board 19a, as shown in FIG. 7, the illuminating board 19a needs to be arranged substantially parallel to the plate-like portion 14a of the positioning unit 14, and the outer circumference of the upper end of the cylindrical lens frame 4d needs to be accurately aligned with the opening part H1. Accordingly, not only alignment of the outer circumference of the upper end of the lens frame 4d with the opening part H1 takes time and labor, but also, as is seen from comparison between FIGS. 6 and 7, a length of the extending part A1 needs to be made longer than the case in which the lens frame 4d having the tapered external shape is inserted into the opening part H1.

That is, because the lens frame 4d has the tapered external shape, the opening part H1 of the illuminating board 19a can be easily aligned with the outer circumference of the upper end E1. As a result, the lens frame 4d can be easily inserted into the opening part H1 of the illuminating board 19a, and the length of the extending part A1 formed on a part of the outer circumference of the illuminating board 19a can be minimized.

Because the lens frame 7d of the optical unit 7 on the direction B side shown in FIG. 1 has a tapered external shape with one end (specifically, an upper end, which is an opening end on the lens 7a side) being tapered, as in the lens frame 4d of the optical unit 4 on the direction F side, the lens frame 7d can be easily inserted into and fixed in the through hole of the plate-like portion 15a of the positioning unit 15 as in the lens frame 4d. Further, the lens frame 7d can be easily inserted into the opening part H2 of the illuminating board 19f by having the tapered shape, and the length of the extending part A5 formed on a part of the outer circumference of the illuminating board 19f can be minimized.

As explained above, in the capsule medical device according to the first embodiment of the present invention, the external shape of the lens frame for holding the lens group that forms images of inside the subject illuminated by the illuminating unit onto the light receiving surface of the imaging unit is formed in a tapered shape, the outer diameter of the tapered end of the lens frame is made smaller than the opening part formed in the illuminating board having the illuminating unit mounted thereon, and the lens frame is inserted into the opening part of the illuminating board from the tapered end. Accordingly, when the lens frame is inserted into the opening part of the illuminating board, the opening part can be easily aligned with the outer circumference of the upper end of the lens frame. As a result, the lens frame can be easily inserted into the opening part of the illuminating board, and the illuminating board and the lens frame can be easily fitted to each other, while avoiding a contact between the upper end of the lens frame and the illuminating board.

The length of the bending part (extending part) extending to a part of the outer circumference of the illuminating board can be also minimized. As a result, downsizing of the device scale can be facilitated, and the board cost required for manufacturing the capsule medical device can be reduced.

Further, in the capsule medical device according to the first embodiment of the present invention, because the flexible board is used as the circuit board such as the illuminating board, the imaging board, and the wireless board, downsizing and weight saving of the capsule medical device can be facilitated and the board cost can be further reduced, as compared to the capsule medical device using the rigid board as the circuit board.

### (Second embodiment)

A second embodiment of the present invention is explained next. In the first embodiment described above, the external shape of the lens frame of the optical unit to be inserted into the opening part of the illuminating board is formed in the tapered shape. However, in the second embodiment, an opening part opened and enlarged toward a distal side with respect to an extending part extending to a part of the outer circumference of the illuminating board is formed in the illuminating board, and the lens frame of the optical unit is inserted into the enlarged opening part.

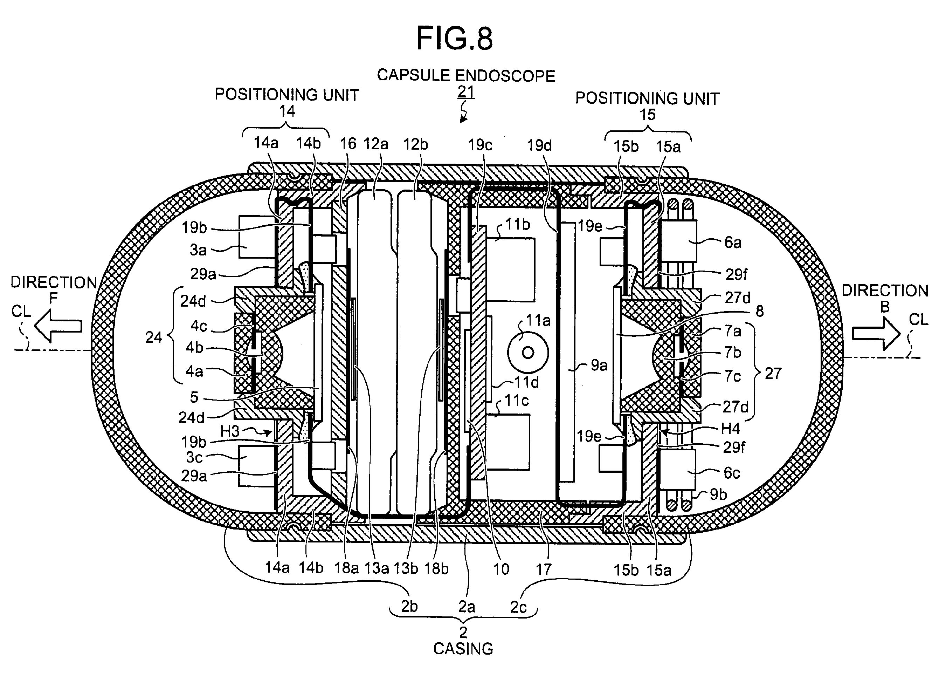

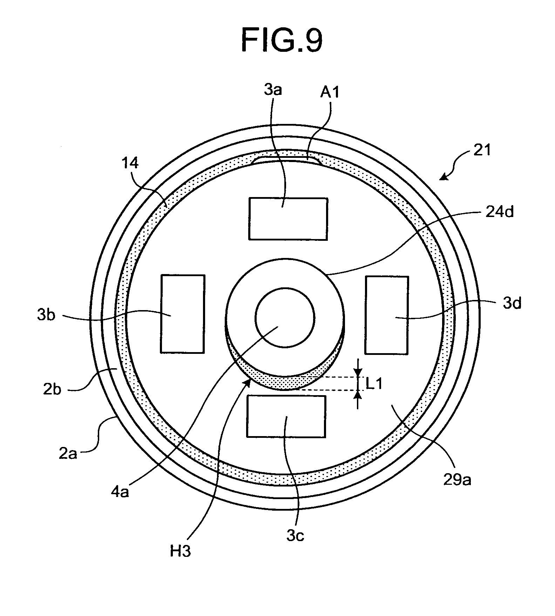

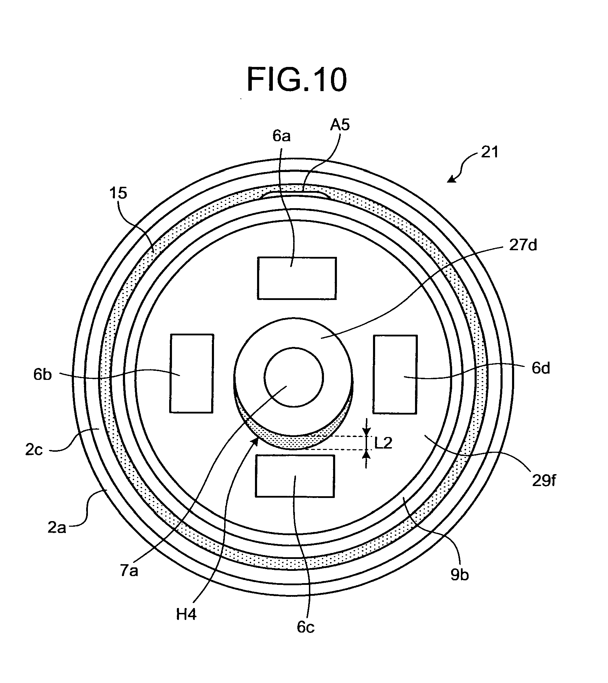

FIG. 8 is a schematic longitudinal cross section of a configuration example of a capsule endoscope according to the second embodiment of the present invention. FIG. 9 is a schematic diagram for exemplifying an internal structure of the capsule endoscope as viewed over an optical dome from a direction F shown in FIG. 8. FIG. 10 is a schematic diagram for exemplifying the internal structure of the capsule endoscope as viewed over the optical dome from a direction B shown in FIG. 8. As shown in FIGS. 8 to 10, a capsule endoscope 21 according to the second embodiment includes optical units 24 and 27 instead of the optical units 4 and 7 of the capsule endoscope 1 according to the first embodiment, and illuminating boards 29a and 29f instead of the illuminating boards 19a and 19f. Other configurations are the same as those in the first embodiment, and like reference characters refer to like parts.

The optical unit 24 includes a lens frame 24d instead of the lens frame 4d having the tapered external shape. The optical unit 24 includes the same configuration as that of the optical unit 4 in the first embodiment, except of the lens frame. Meanwhile, the optical unit 27 includes a lens frame 27d instead of the lens frame 7d having the tapered external shape. The optical unit 27 includes the same configuration as that of the optical unit 7 in the first embodiment, except of the lens frame. The lens frame 27d has a cylindrical external shape, and has the same function and structure as those of the lens frame 7d of the optical unit 7, except of the external shape.

The illuminating board 29a includes an opening part H3 (an opening part having a substantially disk-like shape matched with a body of the lens frame 4d) instead of the opening part H1 of the illuminating board 19a in the first embodiment. The opening part H3 is obtained by further enlarging an aperture having a diameter capable of inserting the lens frame 24d. As shown in FIG. 9, the opening part H3 is formed by enlarging an original aperture matched with the outer diameter of the lens frame 24d by a length L1 toward a distal side with respect to the extending part A1 extending to a part of the outer circumference of the illuminating board 29a. The illuminating board 29a has the same function and structure as those of the illuminating board 19a.

The illuminating board 29f includes an opening part H4 (an opening part having a substantially disk-like shape matched with a body of the lens frame 7d) instead of the opening part H2 of the illuminating board 19f in the first embodiment. The opening part H4 is obtained by further enlarging an aperture having a diameter capable of inserting the lens frame 27d. As shown in FIG. 10, the opening part H4 is formed by enlarging an original aperture matched with the outer diameter of the lens frame 27d by a length L2 toward a distal side with respect to the extending part A5 extending to a part of the outer circumference of the illuminating board 29f. The illuminating board 29f has the same function and structure as those of the illuminating board 19f.

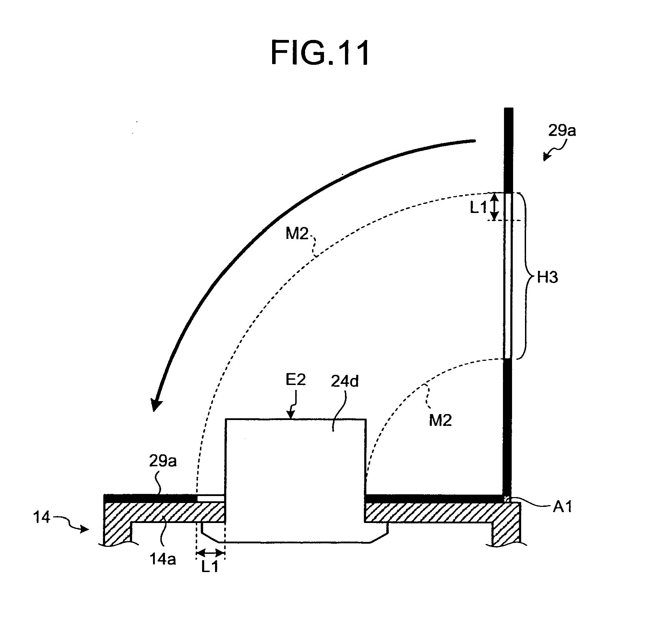

Fitting of the illuminating board 29a and the lens frame 24d is explained next. FIG. 11 is a schematic diagram for explaining fitting of the lens frame 24d fitted to the plate-like portion 14a of the positioning unit 14 and the illuminating board 29a.

As shown in FIGS. 8 and 11, the lens frame 24d of the optical unit 24 has a cylindrical external shape, and is fitted to the plate-like portion 14a of the positioning unit 14. In this case, the lens frame 24d is fitted and fixed in the through hole formed in the plate-like portion 14a. The illuminating board 29a is fitted to the lens frame 24d fitted and fixed to the plate-like portion 14a.

Specifically, as shown in FIG. 11, the illuminating board 29a is arranged on an upper face side (a surface facing the optical dome 2b shown in FIG. 8) of the plate-like portion 14a, continuously with the extending part A1 inserted into a notch (not shown) formed in the positioning unit 14. The extending part A1 functions as a bending part, which enables the illuminating board 29a to turn toward the upper face of the plate-like portion 14a by bending, similarly to the first embodiment.

The illuminating board 29a having the extending part A1 on a part of the outer circumference turns around the extending part A1 in a direction of moving the opening part H3 toward the lens frame 24d, while bending the extending part A1. The illuminating board 29a is fixed to the upper face of the plate-like portion 14a while inserting the lens frame 4d into the opening part H3. Thus, by fixing the illuminating board 29a to the upper face of the plate-like portion 14a, with the lens frame 24d being inserted into the opening part H3, fitting of the lens frame 24d to the illuminating board 29a is complete.

The illuminating board 29a has the opening part H3 formed by enlarging an original aperture (aperture matched with the outer diameter of the lens frame 24d) by the length L1 toward the distal side with respect to the extending part A1. The opening part H3 can avoid a contact between the illuminating board 29a, which turns while bending the extending part A1, and an upper end E2 of the lens frame 24d. In this case, as shown in FIG. 11, the upper end E2 of the lens frame 24d is positioned inside of a locus M2 drawn by a rim of the opening part H3 (that is, the inner circumference of the illuminating board 29a forming the opening part H3), when the illuminating board 29a is turned around the extending part A1 until it is surface-contacted with the plate-like portion 14a. Even if the lens frame 24d has the cylindrical external shape, the illuminating board 29a having the enlarged opening part H3 facilitates alignment between the outer circumference of the upper end E2 of the lens frame 24d and the opening part H3 of the illuminating board 29a, and the lens frame 24d can be easily inserted into the opening part H3, while avoiding a contact between the illuminating board 29a and the outer circumference of the upper end E2. As a result, similarly to in the first embodiment, the length of the extending part A1 formed on a part of the outer circumference of the illuminating board 29a can be minimized.

As in the illuminating board 29a on the direction F side, the illuminating board 29f on the direction B side shown in FIG. 8 has the opening part H4 formed by enlarging the original aperture (an aperture matched with the outer diameter of the lens frame 27d) by the length L2 toward the distal side with respect to the extending part A5. Accordingly, as in the illuminating board 29a, the lens frame 27d can be easily inserted into the opening part H4 and the length of the extending part A5 can be minimized.

As described above, in the capsule medical device according to the second embodiment of the present invention, the opening part is formed in the illuminating board by enlarging the aperture having a diameter capable of inserting the lens frame of the optical unit toward the distal side with respect to the bending part (extending part) formed on the outer circumference of the illuminating board, and the illuminating board is turned around the bending part in the direction of moving the opening part toward the lens frame, thereby inserting the lens frame into the opening part of the illuminating board. Other parts of the configuration are substantially the same as those of the first embodiment. Accordingly, even if the lens frame has the cylindrical external shape, the opening part and the outer circumference of the upper end of the lens frame can be easily aligned with each other at the time of inserting the lens frame into the opening part of the illuminating board. As a result, the lens frame can be easily inserted into the opening part of the illuminating board, and the illuminating board can be easily fitted to the lens frame, while avoiding a contact between the upper end of the lens frame and the illuminating board.

Similarity to the first embodiment, the length of the bending part (extending part) extending to a part of the outer circumference of the illuminating board can be minimized. As a result, downsizing of the device scale can be facilitated, and the board cost required for manufacturing the capsule medical device can be reduced.

Further, because the flexible board is used as the circuit board such as the illuminating board, the imaging board, and the wireless board, downsizing and weight saving of the capsule medical device can be facilitated and the board cost can be further reduced, as compared to the capsule medical device using the rigid board as the circuit board.

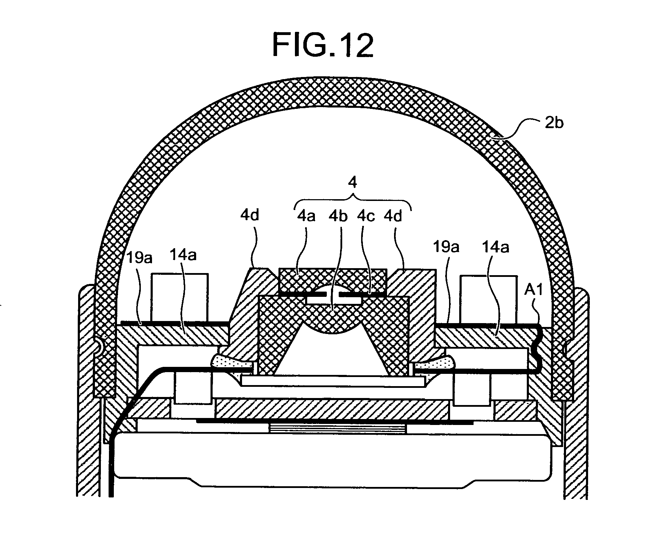

In the first embodiment, the external shape of the lens frames 4d and 7d with one end (upper end) being tapered is in the tapered shape over the entire outer circumference. However, the present invention is not limited thereto, and the lens frames 4d and 7d with one end being tapered may have a tapered external shape only on one side of the outer circumference. Specifically, as shown in FIG. 12, the external shape of the lens frame 4d can be tapered with a distal side with respect to the extending part A1 (bending part) being inclined. In this case, the external shape of the lens frame 4d on the near side with respect to the extending part A1 can be the cylindrical shape and the external shape of the lens frame 4d on the distal side with respect to the extending part A1 can be the tapered shape, designating two straight lines on the outer circumference of the lens frame 4d, equidistant with respect to the extending part A1 as a boundary. The same applies to the lens frame 7d of the optical unit 7.

In the second embodiment, the lens frames 24d and 27d having the cylindrical external shape are inserted into the enlarged opening parts H3 and H4 of the illuminating boards 29a and 29f, respectively. However, the present invention is not limited thereto, and a lens frame having the tapered external shape exemplified by the lens frames 4d and 7d in the first embodiment can be inserted into the enlarged opening parts H3 and H4. That is, the first and second embodiments described above can be combined.

In the first and second embodiments of the present invention, as the capsule medical device introduced into the subject, a capsule endoscope having the imaging function and the wireless communication function, which acquires in-vivo images as an example of the in-vivo information is explained. However, the present invention is not limited thereto, and the capsule medical device can be a capsule pH measuring device that measures pH information in a living body as the in-vivo information, a capsule drug-administering device having a function of spraying or injecting a drug into the living body, or a capsule sampling device that samples a substance in the living body (tissue of the body) as the in-vivo information.

### INDUSTRIAL APPLICABILITY

As described above, the capsule medical device and the method of manufacturing a capsule medical device according to the present invention are useful for a capsule medical device introduced into a subject, and particularly suitable for a capsule medical device in which a lens frame of an optical unit that forms images of inside the subject onto a light receiving surface of an imaging unit can be easily inserted into an opening part of an illuminating board having an illuminating unit that illuminates the inside of the subject mounted thereon, and a method of manufacturing a capsule medical device.

## Claims

1. A capsule medical device introduced into a subject, and including an illuminating unit that illuminates inside of the subject and an imaging unit that captures an image of inside the subject illuminated by the illuminating unit, the capsule medical device comprising:
an optical unit including a lens group that forms the image of inside the subject illuminated by the illuminating unit onto a light receiving surface of the imaging unit and a lens frame for holding the lens group; and
an illuminating board having an opening part for inserting the lens frame thereinto formed thereon and including the illuminating unit mounted thereon, wherein
the lens frame has a tapered external shape with one end being tapered, and is inserted into the opening part of the illuminating board from the one end being tapered.

2. The capsule medical device according to claim 1, wherein
the illuminating board has a bending part extending to a part of an outer circumference, and turns in a direction of moving the opening part toward the lens frame by bending the bending part, and
the lens frame has the tapered external shape capable of being inserted into the opening part while avoiding a contact between the illuminating board, which turns while bending the bending part, and the one end.

3. The capsule medical device according to claim 2, wherein the lens frame has the tapered external shape, with a distal side with respect to the bending part being inclined.

4. A capsule medical device introduced into a subject, and including an illuminating unit that illuminates inside of the subject and an imaging unit that captures an image of inside the subject illuminated by the illuminating unit, the capsule medical device comprising:
an optical unit including a lens group that forms the image of inside the subject illuminated by the illuminating unit onto a light receiving surface of the imaging unit and a lens frame for holding the lens group; and
an illuminating board having a bending part extending to a part of an outer circumference, and an opening part in which an aperture capable of inserting the lens frame of the optical unit thereinto is opened and enlarged toward a distal side with respect to the bending part formed thereon, and having the illuminating unit mounted thereon, wherein
the illuminating board turns in a direction of moving the opening part toward the lens frame by bending the bending part, and
the lens frame is inserted into the opening part while avoiding a contact between the illuminating board, which turns while bending the bending part, and an upper end of the lens frame.

5. A capsule medical device introduced into a subject, and including an illuminating unit that illuminates inside of the subject and an imaging unit that captures an image of inside the subject illuminated by the illuminating unit, the capsule medical device comprising:
an optical unit including a lens group that forms the image of inside the subject illuminated by the illuminating unit onto a light receiving surface of the imaging unit and a lens frame for holding the lens group; and
an illuminating board having a bending part extending to a part of an outer circumference and an opening part capable of inserting the lens frame thereinto formed thereon, and having the illuminating unit mounted thereon, wherein
at least one of the opening part and the lens frame has a shape capable of avoiding a contact between the illuminating board, which turns while bending the bending part, and an upper end of the lens frame.

6. The capsule medical device according to claim 5, wherein the shape capable of avoiding the contact is a tapered external shape with one end of the lens frame being tapered.

7. The capsule medical device according to claim 5, wherein the shape capable of avoiding the contact is an opening shape in which the opening part of the illuminating board is opened and enlarged toward a distal side with respect to the bending part.

8. A capsule medical device introduced into a subject, and including an illuminating unit that illuminates inside of the subject and an imaging unit that captures an image of inside the subject illuminated by the illuminating unit, the capsule medical device comprising:
an optical unit including a lens group that forms the image of inside the subject illuminated by the illuminating unit onto a light receiving surface of the imaging unit and a lens frame for holding the lens group; and
an illuminating board having a bending part extending to a part of an outer circumference and an opening part capable of inserting the lens frame thereinto formed thereon, and having the illuminating unit mounted thereon, wherein
the illuminating board, which turns while bending the bending part, turns in a direction of moving the opening part toward an upper end of the lens frame, and
the capsule medical device has a shape capable of inserting the lens frame into the opening part while avoiding a contact between the opening part and the upper end of the lens frame.

9. The capsule medical device according to claim 8, wherein
the shape capable of inserting the lens frame into the opening part while avoiding the contact is formed of:
the lens frame having a tapered external shape with an upper end being tapered; and
the opening part of the illuminating board having an opening shape opened and enlarged toward a distal side with respect to the bending part.

10. The capsule medical device according to any one of claims 1 to 9, wherein the illuminating board is a flexible circuit board.

11. A method of manufacturing a capsule medical device comprising:
a step of bending an illuminating board having an opening part for inserting a lens frame thereinto formed thereon, and a bending part extending to a part of an outer circumference thereof; and
a step of inserting the lens frame having a tapered external shape with one end being tapered into the opening part while avoiding a contact between the illuminating board and the lens frame.

12. A method of manufacturing a capsule medical device comprising:
a step of bending an illuminating board having a bending part extending to a part of an outer circumference thereof and an opening part, which is opened and enlarged toward a distal side with respect to the bending part, for inserting a lens frame thereinto; and
a step of inserting the lens frame into the opening part while avoiding a contact between the illuminating board and the lens frame.
